# FASCICULE DE BREVET EUROPEEN

(11) **EP 0 804 399 B1**
(45) Date de publication et mention de la délivrance du brevet: **27.12.2000**
(21) Numéro de dépôt: 96901399.4
(22) Date de dépôt: 16.01.1996
(51) Int. Cl.: C07C 45/50, C07C 45/49, C07C 67/293, C07C 67/347

(54) **PROCEDE D'HYDROFORMYLATION D'UNE OLEFINE EN MILIEU BIPHASIQUE**
VERFAHREN ZUR HYDROFORMYLIERUNG EINES OLEFINS IN EINEM BIPHASISCHEN MEDIUM
METHOD FOR OLEFIN HYDROFORMYLATION IN A BIPHASIC MEDIUM

(30) Priorité: 17.01.1995 FR 9500466
(43) Date de publication de la demande: 05.11.1997
(73) Titulaire: CENTRE NATIONAL DE LA RECHERCHE SCIENTIFIQUE (CNRS), 75016 Paris (FR)
(72) Inventeur: MONFLIER, Eric, F-59000 Lille (FR); MORTREUX, André, F-59510 Hem (FR); CASTANET, Yves, F-59510 Hem (FR)
(74) Mandataire: Sueur, Yvette
(86) Numéro de dépôt international: FR9600069
(87) Numéro de publication internationale: WO9622267

(56) Documents cités:
- EP-A- 0 157 316
- FR-A- 2 489 308
- ANGEWANDTE CHEMIE. INTERNATIONAL EDITION, vol. 34, no. 20, 3 Novembre 1995, pages 2269-2271, XP000535260 MONFLIER E ET AL: "MOLECULAR RECOGNITION BETWEEN CHEMICALLY MODIFIED BETA-CYCLODEXTRIN AND DEC-1-ENE: NEW PROSPECTS FOR BIPHASIC HYDROFORMYLATION OF WATER-INSOLUBLE OLEFINS"
- TETRAHEDRON LETT. (TELEAY,00404039);95; VOL.36 (52); PP.9481-4, UNIV. ARTOIS;FAC. SCIENCES J. PERRIN; LENS; 62307; FR. (FR), XP002003553 MONFLIER E ET AL: "A further breakthrough in biphasic rhodium-catalyzed hydroformylation: use of per(2,6-di-O-methyl)-.beta.-cyclodextrin as inverse phase transfer catalyst"
- CATAL. LETT. (CALEER,1011372X);91; VOL.9 (1-2); PP.55-8, MONASH UNIV.;DEP. CHEM.; CLAYTON; 3168; AUSTRALIA (AU), XP002003554 ANDERSON J R ET AL: "Hydroformylation of olefins with water-soluble rhodium catalysts in the presence of.alpha.-cyclodextrin" cité dans la demande

## Description

La présente invention concerne un procédé pour l'hydroformylation d'une oléfine.

Il est connu que l'hydroformylation des oléfines peut être effectuée en milieu homogène en présence de solvants avec un très bon rendement. Toutefois, l'addition de solvants complique le procédé en particulier dans le cas d'oléfines à masse molaire élevée. En effet, dans un tel milieu, les aldéhydes ne peuvent être séparées du catalyseur que par une distillation du mélange réactionnel brut. Dans le cas d'oléfines lourdes, compte tenu des températures d'ébullition des oléfines et des produits formés, cette distillation doit être effectuée à des températures élevées qui provo.quent à moyen ou long terme une dégradation importante du catalyseur, ce qui est économiquement inacceptable en particulier lorsque le catalyseur est à base de métaux nobles (Rh, Pt ...).

Pour supprimer cet inconvénient, on a effectué l'hydroformylation des oléfines supérieures par CO/H₂ en milieu biphasique. Le but était de permettre la séparation aisée du catalyseur (se trouvant dans une phase) des produits obtenus (présents dans l'autre phase) en effectuant une simple décantation. Deux grands types de systèmes biphasiques ont été décrits : le système eau-phase organique (le plus courant) et, plus récemment, le système perfluoroalcane-phase organique.

Différentes solutions ont été proposées pour solubiliser le catalyseur dans les perfluoroalcanes ou dans l'eau, consistant essentiellement à utiliser des phosphines fluorées dans le premier cas et hydrosolubles dans le second.

Ainsi, I. T. Horvath et al, (Science 1994 Vol. 266, 72) décrivent l'hydroformylation du 1-décène en solution dans le toluène, catalysée par le complexe HRh(CO)[P{CH₂CH₂(CF₂)₅CF₃}₃]₃ qui est dissout dans C₆F₁₁CF₃ et en présence de P{CH₂CH₂(CF₂)₅CF₃}₃. Dans ce procédé, le catalyseur est facilement recyclable car il se trouve dans la phase perfluorée, alors que l'aldéhyde se trouve dans la phase toluénique non miscible avec la précédente. La principale difficulté réside toutefois dans la synthèse de la phosphine très particulière utilisée. D'autre part la quantité de rhodium présente dans la phase toluénique n'est pas précisée.

L'utilisation de systèmes biphasiques eau-phase organique est beaucoup plus fréquente. Ainsi E. Kuntz (Rhone-Poulenc) a proposé d'utiliser la triphénylphosphine trisulfonée (TPPTS) pour solubiliser des complexes métalliques dans l'eau. Cette technique a été mise en oeuvre industriellement, en particulier pour réaliser l'hydroformylation du propène en butanal avec des systèmes catalytiques à base de rhodium et de TPPTS en solution aqueuse (voir par exemple Angewandte Chemie, Int. Ed. Eng. 1993, 1524-1544).

L'utilisation d'une phosphine hydrosoluble améliore certes le procédé d'hydroformylation, mais uniquement dans le cas d'oléfines légères partiellement solubles en milieu aqueux, le rendement étant très faible avec les oléfines grasses.

Aussi diverses études ont visé à augmenter la solubilité des oléfines grasses en phase aqueuse de manière à pouvoir leur appliquer le procédé biphasique. Les solutions proposées consistaient principalemqnt à ajouter un agent tensioactif au milieu réactionnel contenant un complexe de rhodium, une phosphine hydrosoluble et l'oléfine.

Par exemple dans US-A-4399312, 1981, Russel décrit l'utilisation comme agent tensioactif de bromure de dodécyl-triméthylammonium ayant pour but de rendre une oléfine grasse, le 1-décène, soluble en milieu aqueux. Le rendement reste toutefois encore modeste. Dans WO 93/04029, le même système catalytique a été utilisé, mais en récupérant le catalyseur par filtration sur membrane, ce qui permet d'aboutir à des concentrations résiduelles en rhodium extrêmement faibles dans la phase organique. Néanmoins, cette technique complique beaucoup la récupération du catalyseur.

Hanson et al, (J. Mol. Catal. 1994 Vol. 88 43-56), décrit l'utilisation de dodécylbenzènesulfonate de sodium en présence de différentes phosphines hydrosolubles pour l'hydroformylation de l'octène. Les rendements en aldéhyde ne dépassaient cependant pas 80 % pour la phosphine la plus performante.

L'utilisation de sel d'ammonium ou de sel de phosphonium comme agent tensioactif pour l'hydroformylation d'oléfines grasses est décrite dans EP-A-0602444 et EP-A-0602463. L'utilisation d'un sel de rhodium, de la TTPTS comme phosphine hydrosoluble et du dodécylsulfate de sodium comme agent tensioactif, en présence de butanol pour réaliser une micro-émulsion, est décrite dans EP-380154 (Eniricerche).

Dans tous ces procédés, l'introduction d'un agent tensioactif a pour résultat la formation d'une émulsion stable qui rend très difficile, voire impossible, la séparation de la phase organique qui contient le catalyseur, de la phase aqueuse qui contient l'aldéhyde.

D'autre part, les cyclodextrines sont des oligomères cycliques du glucose pontés en α (1-4). Dans ce type de composé, le nombre d'unités α-glucosiques atteint respectivement 6, 7 et 8 pour l'α-cyclodextrine, la β-cyclodextrine et la γ-cyclodextrine. Leur mise en oeuvre dans des réactions de catalyse homogène biphasique a permis d'améliorer la vitesse et la sélectivité de certaines réactions. Ainsi, H. Alper et al. a réalisé l'oxydation d'oléfines en cétones (J. Mol. Catal. 1986 Vol. 35 p. 249) en utilisant la β-cyclodextrine comme agent de transfert de phase. L'oxydation d'alcènes en cétones en milieu biphasique, catalysée par un système constitué de palladium / cyclodextrine a aussi été décrite par E. Monflier, et al. (Angew. Chemie 1994,106, 20, 2183). Les auteurs ont constaté que la présence d'une cyclodextrine, substituée ou non substituée, améliorait notablement le rendement de l'oxydation. L'hydroformylation d'oléfines en présence d'α-cyclodextrine a été décrite par R. Anderson et al. (Catalysis Letters, 1991 Vol. 9 p. 55). Différents complexes de métaux de transition ont été utilisés comme catalyseurs, notamment des complexes de rhodium, ainsi que différentes phosphines. Les résultats obtenus à la suite d'essais comparatifs effectués en présence d'α-cyclodextrine non modifiée, ont amené les auteurs à conclure que l'addition de cyclodextrine au mélange réactionnel avait un effet inhibiteur sur l'hydroformylation des alcènes.

Les présents inventeurs ont maintenant trouvé que, de manière surprenante, l'utilisation d'une cyclodextrine modifiée à la place d'une cyclodextrine non modifiée, non seulement n'avait pas d'effet inhibiteur sur la réaction d'hydroformylation, mais améliorait substantiellement le rendement de l'hydroformylation par rapport au procédé n'utilisant pas de cyclodextrine.

C'est pourquoi la présente invention a pour objet un procédé d'hydroformylation d'une oléfine choisie parmi les alcènes représentés par la formule CₙH₂ₙ dans laquelle n est un nombre entier inférieur ou égal à 30 ou parmi les oléfines fonctionnalisées, par un mélange gazeux CO/H₂ en milieu biphasique, consistant à mettre en contact d'une part une solution aqueuse contenant un système catalytique constitué par un complexe de métal de transition hydrosoluble et une phosphine hydrosoluble, et d'autre part une oléfine, à chauffer sous agitation le mélange réactionnel obtenu après l'avoir mis sous une pression de mélange gazeux CO/H₂, le dit procédé étant caractérisé en ce que l'on introduit une cyclodextrine modifiée dans la solution aqueuse contenant le système catalytique, ladite cyclodextrine modifiée étant choisie parmi les α-cyclodextrines, les β-cyclodextrines et les γ-cyclodextrines constituées respectivement de 6, 7 et 8 unités glucosiques et portant un ou plusieurs substituants identiques ou différents, choisis parmi les radicaux alkyles linéaires ou ramifiés ayant de 1 à 20 atomes de carbone, les radicaux hydroxyalkyles ayant de 1 à 20 atomes de carbone, les radicaux carboxyles, amino, phosphates, éthers, polyéthers, les radicaux comprenant une fonction ester ayant de 1 à 20 atomes de carbone, ou parmi les cyclodextrines sous forme polymérisée avec l'épichlorhydrine.

Les alcènes qui peuvent être hydroformylés par le procédé de l'invention peuvent être linéaires ou ramifiés. Les alcènes ayant de 8 à 20 atomes de carbone sont préférés, en particulier les alcènes linéaires ayant de 8 à 16 atomes de carbone, plus spécialement les alcènes ayant une double liaison en position terminale. A titre d'exemple de tels alcènes, on peut citer le 1-heptène, le 1-octène, le 1-nonène, le 1-décène, le 1-undécène, le 1-dodécène, le 1-tridécène et le 1-tétradécène.

Dans le présent texte, une oléfine fonctionnalisée désigne un alcène linéaire ou ramifié ayant un encombrement inférieur au diamètre d'entrée de la cyclodextrine utilisée et dans lequel au moins l'un des atomes de carbone de la double liaison éthylénique porte au moins un substituant choisi parmi :
- les radicaux alicycliques, saturés ou insaturés, de préférence ceux qui ont de 6 à 30 atomes de carbone ;
- les radicaux alcényles linéaires ou ramifiés, de préférence ceux qui ont de 2 à 30 atomes de carbone ;
- les radicaux aromatiques comprenant un ou plusieurs noyaux aromatiques non condensés non substitués ou substitués par un radical alkyle (ayant de préférence au plus trois atomes de carbone) ou par un groupement fonctionnel, tel que par exemple une fonction ester, une fonction nitrile, une fonction cétone, une fonction acétal ou une fonction hydroxyle ;
- les radicaux aromatiques comprenant un ou plusieurs noyaux aromatiques condensés, non substitués ou substitués par un radical alkyle (ayant de préférence au plus trois atomes de carbone) ou par un groupement fonctionnel, tel que par exemple une fonction ester, une fonction nitrile, une fonction cétone, une fonction acétal ou une fonction hydroxyle, de préférence les radicaux naphtyles portant éventuellement un ou plusieurs substituants alkyle ayant de 1 à 3 atomes de carbone ;
- les radicaux alkyles portant un substituant aryle ou un groupement fonctionnel, tel que par exemple une fonction ester, une fonction nitrile, une fonction cétone, une fonction acétal ou une fonction hydroxyle ;
- les fonctions ester et les fonctions acyloxy.

Parmi les oléfines fonctionnalisées, on préfère celles dans lesquelles la double liaison carbone-carbone porte au moins un atome d'hydrogène et plus spécialement au moins deux atomes d'hydrogène. Les oléfines fonctionnalisées dans lesquelles la double liaison carbone-carbone porte trois atomes d'hydrogène sont particulièrement préférées.

Le procédé peut être mis en oeuvre pour une oléfine ayant une seule double liaison, en position terminale ou non, ou pour une oléfine ayant plusieurs doubles liaisons. Il permet d'hydroformyler une oléfine seule, ou un mélange de différentes oléfines.

Le procédé de la présente invention est particulièrement intéressant pour l'hydroformylation d'un alcène linéaire ou ramifié ayant de 2 à 30 atomes de carbone, plus spécialement pour les alcènes ayant de 8 à 30 atomes de carbone, qui ne peuvent être hydroformylés avec un bon rendement par les procédés de l'art antérieur.

Le catalyseur utilisé dans le procédé de la présente invention est un complexe d'un métal de transition choisi parmi le rhodium, le ruthénium, le palladium, l'iridium, le cobalt, le platine, le couple platine/étain, le couple platine/fer, avec un ligand phosphoré hydrosoluble. Les complexes du rhodium sont particulièrement préférés. A titre d'exemples non limitatifs, on peut citer Rh₆(CO)₁₆, Rh₄(CO)₁₂, HRhCO(PR₃)₃ et RhCl(PR₃)₃ (dans lesquels R représente un groupement aryle sulfoné, de préférence un groupement phényle sulfoné), Rh(acac)(CO)₂ (acac représentant un radical acétylacétonate), Rh₂Cl₂(CO)₄, les oxydes de rhodium (Rh₂O₃) et les sels de rhodium tels que RhCl₃, Rh(OAc)₃.

Les phosphines hydrosolubles répondent à la formule générale suivante : 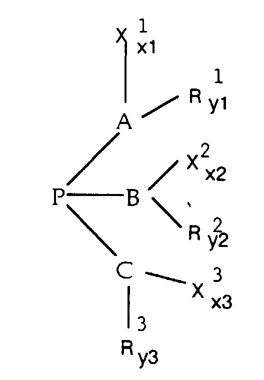 dans laquelle :
- A, B, C, qui peuvent être identiques ou différents, représentent chacun un groupement aryle choisi parmi les groupements phényles et les groupements naphtyles, ou parmi les groupements alkyles ayant de 1 à 10 atomes de carbone ;
- les substituants R¹, R², R³, qui peuvent être identiques ou différents, représentent chacun un groupement choisi parmi l'hydrogène, les radicaux alkyles ayant de 1 à 4 atomes de carbone, les radicaux alkoxy ayant de 1 à 4 atomes de carbone, les atomes d'halogène, les groupes -CN,-NO₂, -OH et -N(Z¹)(Z²), Z¹ et Z², identiques ou différents, représentant chacun un radical alkyle ayant de 1 à 5 atomes de carbone ;
- les substituants X¹, X² et X³, qui peuvent être identiques ou différents, représentent chacun un groupement acide carboxylique, un groupement acide sulfonique ou leurs sels. Lorsque X¹, X² ou X³ représentent un sel d'acide carboxylique ou sulfonique, le cation peut être un cation de métal alcalin ou alcalino-terreux ou un cation ammonium de formule N(T¹T²T³T⁴)⁺ dans laquelle T¹, T², T³, T⁴, qui peuvent être identiques ou différents, représentent chacun un groupe alkyle comportant de 1 à 4 atomes de carbone.
- x1, x2, x3, sont des nombres identiques ou différents, variant entre 0 et 3 inclus, l'un au moins parmi eux étant égal ou supérieur à 1 ;
- y1, y2, y3 sont des nombres identiques ou différents variant entre 2 et 5 inclus.

De préférence, A, B et C représentent chacun un groupement aryle, plus particulièrement un groupement phényle ; les substituants X¹ à x³ sont -SO₃Na ou -CO₂Na ; les substituants R¹ à R³ sont l'hydrogène ou un groupement alkyle ; les nombres x1, x2 et x3 sont égaux à 1 ou 0 (l'un d'entre eux au moins étant égal à 1) ; les nombres y1, y2 et y3 sont égaux à 4 ou 5. On peut citer à titre d'exemples, la triphénylphosphine trisulfonée (TPPTS) (A = B = C = phényle ; R¹ = R² = R³ = H ; X¹ = X² = X³ = SO₃Na ; x1 = x2 = x3 = 1 ; y1 = y2 = y3 = 4), la triphénylphosphine monosulfonée (TPPMS), la triphénylphosphine monocarboxylique (TPPMC) et la triphénylphosphine tricarboxylique (TPPTC). Les phosphines particulièrement avantageuses pour le procédé de la présente invention sont la TPPTS et la TPPMS.

La présente invention peut aussi être mise en oeuvre avec une diphosphine hydrosoluble de formule générale: 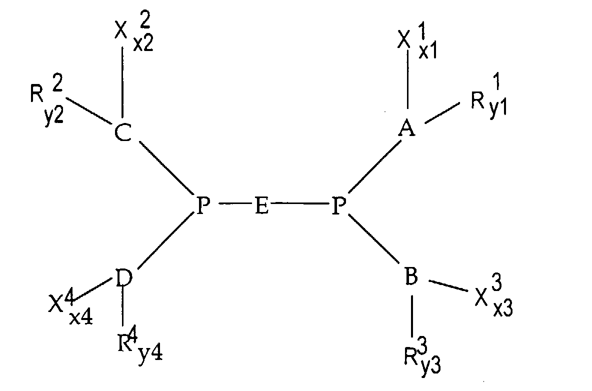 dans laquelle :
- A, B, C, D, qui peuvent être identiques ou différents, représentent chacun un groupement aryle choisi parmi les groupements phényles ou naphtyles, ou un groupement alkyle ayant de 1 à 10 atomes de carbone ;
- E représente une chaîne alkyle linéaire ou ramifiée comportant de 1 à 10 atomes de carbone ou une chaîne comportant un ou plusieurs groupements phényles substitués ou non, ou un cycloalcane comprenant éventuellement des substituants alkyles linéaires ou ramifiés ayant de 1 à 6 atomes de carbone.
- les substituants R¹, R², R³ et R⁴ qui peuvent être identiques ou différents, représentent chacun un groupement choisi parmi l'hydrogène, les radicaux alkyles ayant de 1 à 4 atomes de carbone, les radicaux alkoxy ayant de 1 à 4 atomes de carbone, les atomes d'halogène, les groupes -CN, -NO₂, -OH et -N(Z¹)(Z²), Z¹ et Z² identiques ou différents représentant chacun un radical alkyle ayant de 1 à 5 atomes de carbone ;
- les substituants X¹, X², X³ et X⁴, qui peuvent être identiques ou différents, représentent chacun un groupement acide carboxylique, un groupement acide sulfonique ou leurs sels. Lorsque X¹, X², X³ ou X⁴ représentent un sel d'acide carboxylique ou sulfonique, le cation peut être un cation de métal alcalin ou alcalino-terreux ou un cation ammonium de formule N(T¹T²T³T⁴)⁺ dans laquelle T¹, T², T³, T⁴, qui peuvent être identiques ou différents, représentent chacun un groupe alkyle comportant de 1 à 4 atomes de carbone.
- x1, x2, x3 et x4 sont des nombres identiques ou différents, variant entre 0 et 3 inclus, l'un au moins parmi eux étant égal ou supérieur à 1 ;
- y1, y2, y3 et y4 sont des nombres identiques ou différents variant entre 2 et 5 inclus.

De préférence, A, B, C, D sont des groupements aryles, en particulier un groupement phényle ; E est une chaîne alkyle linéaire comportant 2 à 4 atomes de carbone ; les substituants X¹ à X⁴ sont -SO₃Na et -CO₂Na ; les substituants R¹ à R⁴ sont l'hydrogène ou un groupement alkyle ; les nombres x1 à x4 sont égaux à 1 ou 0 (l'un d'entre eux au moins étant égal à 1) ; les nombres y1 à y4 sont égaux à 4 ou 5. A titre d'exemple, on peut citer la 1,2-bis(diphénylphosphino)éthane sulfonée et la 1,3-bis(diphénylphosphino)-propane sulfonée.

Une cyclodextrine modifiée utilisée dans le procédé de la présente invention est une cyclodextrine qui porte un ou plusieurs substituants identiques ou différents. Comme substituant alkyle, on préfère un radical alkyle linéaire ou ramifié ayant de 1 à 5 atomes de carbone, plus particulièrement un radical méthyle ou éthyle. Comme substituant hydroxyalkyle, on préfère un groupe hydroxyalkyle ayant de 1 à 5 de carbone, plus particulièrement un radical -CH₂CH₂OH ou -CH₂OH. Comme radical contenant une fonction ester, on préfère un groupement ester ayant de 1 à 5 de atomes de carbone, plus particulièrement les groupements -O(CO)CH₃, -O(CO)CH₂CH₃. La cyclodextrine modifiée peut être une α-cyclodextrine modifiée (6 unités glucosiques modifiées par un substituant précédent), dont le diamètre d'entrée est de 4,9 Å, une β--cyclodextrine modifiée (7 unités glucosiques modifiées) dont le diamètre d'entrée est de 6,2 Å, ou une γ-cyclodextrine modifiée (8 unités glucosiques modifiées) dont le diamètre d'entrée est de 7,9 Å. On peut en outre utiliser un mélange de deux ou plusieurs cyclodextrines modifiées mentionnées ci-dessus. Les cyclodextrines modifiées peuvent éventuellement être utilisées sous forme polymérisée avec par exemple l'épichlorhydrine. A titre d'exemple, on peut citer les cyclodextrines méthylées, éthylées, propylées, succinylées, carboxylées, acétylées, 2-hydroxypropylées, polyoxyéthylées. Une cyclodextrine particulièrement avantageuse pour le procédé de la présente invention est la diméthyl-β-cyclodextrine.

La cyclodextrine modifiée peut être introduite dans la solution aqueuse contenant le système catalytique avant l'addition de l'oléfine. Elle peut également être introduite dans ladite solution après l'addition de l'oléfine.

Dans le mélange réactionnel, le rapport molaire phosphine/métal de transition est compris entre 1 et 100, de préférence entre 2 et 20, plus particulièrement égal à 5. Le rapport molaire cyclodextrine modifiée au métal de transition est compris entre 1 et 100, de préférence entre 2 et 20, plus particulièrement égal à 7.

Les conditions de température et de pression sont celles employées classiquement dans les procédés d'hydroformylation biphasique de l'art antérieur. La température est avantageusement comprise entre 20° C et 200° C. La pression est comprise entre 1 et 300 bar, de préférence entre 2 et 100 bar, la pression partielle de monoxyde de carbone étant comprise entre 0,5 et 95 bar. De manière classique, le gaz de synthèse peut être utilisé comme source commode de monoxyde de carbone et d'hydrogène.

La réaction est effectuée en l'absence de solvant pour faciliter le recyclage du catalyseur. En fin de réaction, le catalyseur se trouve dans la phase aqueuse et la séparation entre la phase aqueuse et la phase organique contenant l'aldéhyde est aisée. Toutefois, il est possible d'ajouter un solvant organique non miscible à la phase aqueuse et/ou un solvant polaire non miscible à la phase organique, si nécessaire.

La présente invention est décrite plus en détail dans les exemples donnés ci-dessus, étant entendu que l'invention n'est pas limitée aux exemples décrits.

### Exemple 1

Dans un autoclave de 100 ml, on a introduit 45 ml d'une solution aqueuse contenant 0,8 mmol (426 mg) de TPPTS, 0,16 mmol (41 mg) de Rh(acac) (CO)₂ et 2,24 mmol de diméthylβ-cyclodextrine. On a ensuite ajouté la phase organique constituée par 80 mmol de 1-décène. L'autoclave est chauffé à 80° C, mis sous une pression de 50 bar avec un mélange équimolaire CO/H₂ et l'agitation portée à 1000 tours/minute. La pression a été maintenue constante à l'aide d'un ballast pendant toute la durée de la réaction. La conversion a été suivie par prises d'échantillons. L'analyse chromatographique de ceux-ci (undécane utilisé comme étalon interne) a montré que la conversion était totale au bout de huit heures et que la sélectivité en produits d'hydroformylation était de 95 %. Le rapport molaire undécanal / 2-méthyldécanal était de 1,9. L'activité initiale du catalyseur (nombre de moles de 1-décène transformé par mole de catalyseur et par h) était de 300 h⁻¹.

### Exemples 2 à 10

Ces exemples ont été réalisés dans des conditions expérimentales identiques à celles de l'exemple 1, sauf indication contraire, avec un rapport cyclodextrine / rhodium = 7.

Ils montrent l'influence de la nature du groupement modifiant de la cyclodextrine et des rapports phosphine / rhodium et cyclodextrine / rhodium sur la conversion et la sélectivité. L'exemple 2 est un exemple comparatif sans cyclodextrine et est donné à titre indicatif. Les exemples 9 et 10 montrent que la présente invention peut être mise en oeuvre avec d'autres phosphines hydrosolubles telles que la triphénylphosphine monosulfonée (TPPMS).

Les conditions expérimentales particulières et les résultats obtenus sont rassemblés dans le tableau 1 ci-dessous.

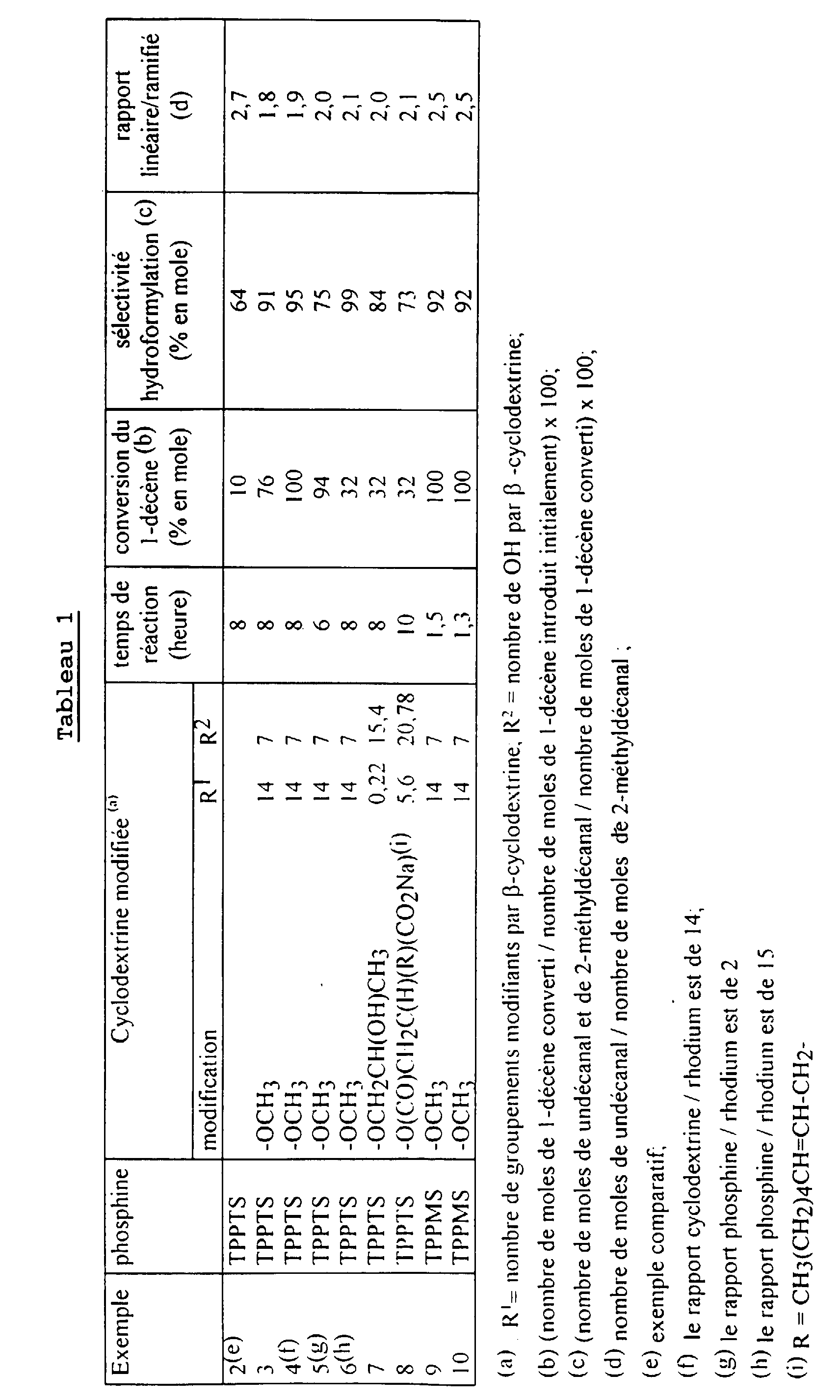

### Exemple 11

Dans un autoclave de 100 ml, on a introduit 45 ml d'une solution aqueuse contenant 0,8 mmol de Co₂(CO)₆(TPPTS)₂ et 0,93 mmol diméthyl-β-cyclodextrine (1,22 g). On a ensuite ajouté la phase organique constituée par 80 mmol de 1-décène. L'autoclave est chauffé à 160° C, mis sous une pression de 50 bar avec un mélange équimolaire CO/H₂ et l'agitation portée à 1000 tours/minute. La pression a été maintenue constante à l'aide d'un ballast pendant toute la durée de la réaction. La conversion a été suivie par prises d'échantillons. L'analyse chromatographique de ceux-ci a montré que la conversion était totale au bout de 18 heures et que la sélectivité en produits d'hydroformylation était de 30 %. Le rapport molaire undécanal / 2-méthyldécanal était de 1,6.

Dans tous ces exemples, la séparation entre les phases organiques et aqueuses est très facile. Il n'y a pas formation d'émulsion et la séparation est très rapide. L'analyse de la phase organique obtenue dans l'exemple 1 montre que la concentration en rhodium y est inférieure à 0,5 ppm et que la concentration en phosphore est de 1,4 ppm ce qui confirme le grand intérêt du procédé. Ainsi, le procédé d'hydroformylation de la présente invention présente un grand intérêt car il permet d'obtenir des aldéhydes gras en recyclant facilement le catalyseur sans perte, ce qui en fait un procédé économique. Les aldéhydes gras sont généralement hydrogénés en alcools gras qui ont de nombreuses applications dans l'industrie chimique, notamment la synthèse de détergents ou la préparation de plastifiants.

### Exemples 12 à 22

Les exemples 12 à 22 suivants illustrent la mise en oeuvre du procédé de l'invention pour diverses oléfines dans des conditions analogues à celles de l'exemple 1, avec un rapport diméthyl-β-cyclodextrine / rhodium = 14 et un rapport TPPTS/Rh = 5.

Les résultats obtenus sont donnés dans le tableau 2 ci-dessus dans lequel :
- les exemples selon l'invention sont numérotés respectivement de 12 à 22 et les résultats obtenus concernant la conversion (Conv.), la sélectivité (Séléc.) et le rapport aldéhyde linéaire/aldéhyde ramifié (lin/ram) sont donnés ;
- chacun des nombres indiqués correspondant aux résultats obtenus par le procédé de l'invention est suivi par un nombre entre parenthèses qui correspond au résultat obtenu par un exemple comparatif réalisé dans les mêmes conditions que l'exemple selon l'invention, mais sans utiliser de diméthyl-β-cyclodextrine ;
- la durée, exprimée en heures, est le temps requis pour obtenir le taux de conversion correspondant indiqué ;
- la conversion représente le rapport (nombre de moles d'oléfine introduite) / (nombre de moles d'oléfine converties) ;
- la sélectivité représente le rapport (nombre de moles d'aldéhyde formées) / (nombre de moles d'oléfine converties) ;
- le rapport (aldéhyde linéaire / aldéhyde ramifié) signifie, pour l'exemple 22, le rapport de β-aldéhyde / α-aldéhyde. L'aldéhyde-y est détecté pour l'exemple 22' comparatif.
- dans l'exemple 15, la double liaison interne n'est pas hydroformylée.

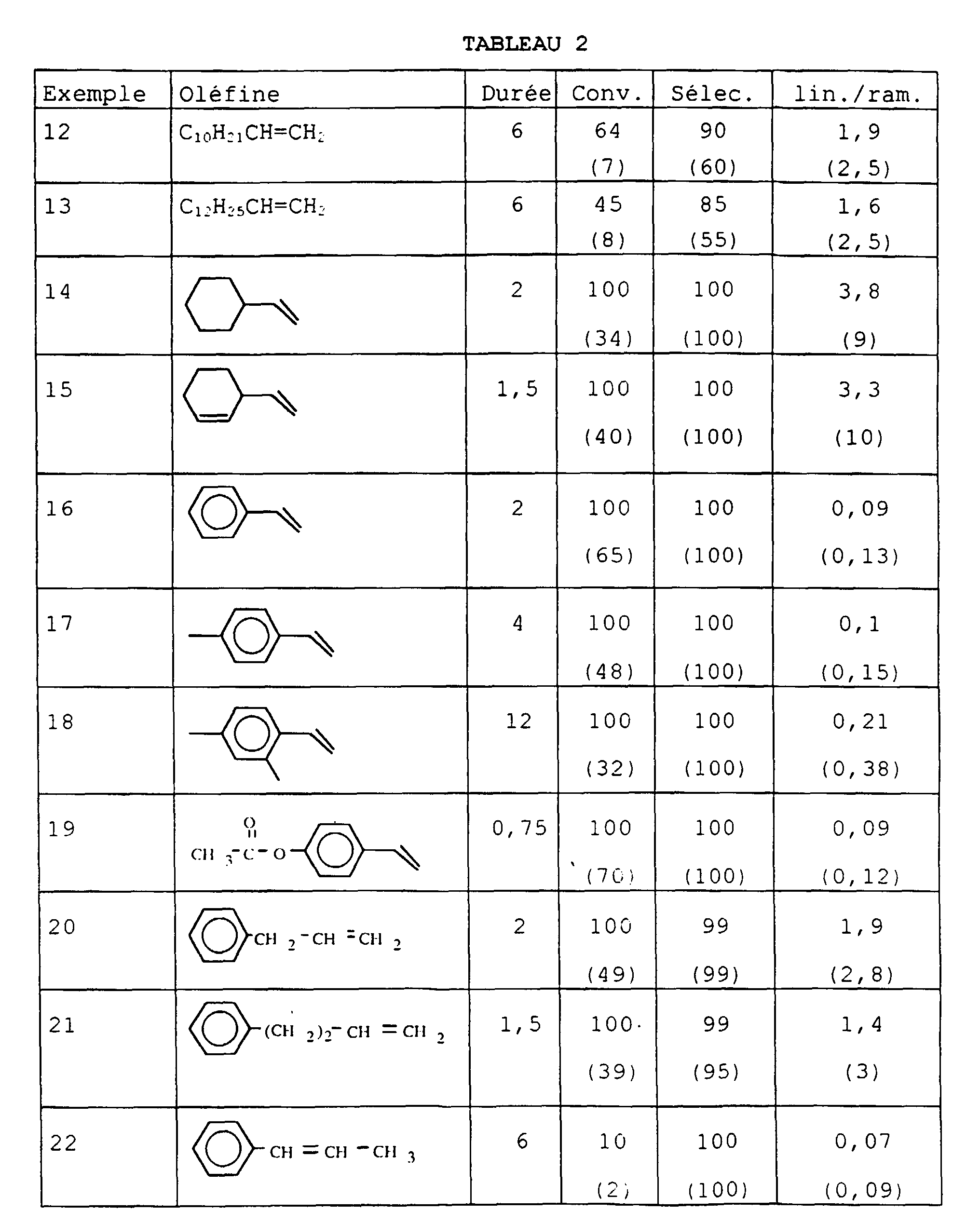

Dans l'exemple 15, la double liaison interne n'est pas formylée.

Les résultats représentés dans le tableau 2 font apparaître clairement l'effet très favorable de la diméthyl β-cyclodextrine sur le taux de conversion de l'oléfine.

### Exemples 23 à 26

Ces exemples illustrent l'hydroformylation de l'undécènoate de méthyle en présente de diméthyl-β-cyclodextrine.

L'undécènoate de méthyle, qui est à la base de la synthèse du Nylon 11, est obtenu industriellement à partir d'un produit naturel, l'huile de ricin, et plus particulièrement par pyrolyse du ricinoléate de méthyle. Par hydroformylation, l'undécènoate de méthyle donne le 10-formyl undécanoate qui, par oxydation ou amination réductrice, peut conduire respectivement à l'acide 1,12-dodécanedioïque précurseur du Nylon 6-12, ou à l'acide α,ω-aminododécanoïque précurseur du Nylon 12.

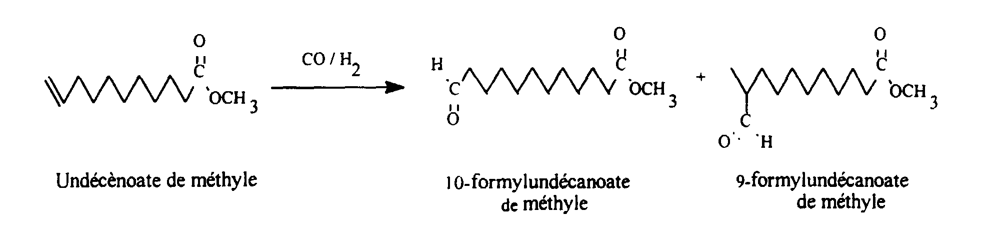

L'exemple 23 illustre l'hydroformylation de l'undécènoate de méthyle, qui a été effectuée dans les conditions suivantes.

Dans un autoclave de 25 ml, on a introduit 11 ml d'une solution aqueuse renfermant 10,3 mg de Rh(acac) (CO)₂ (4.10⁻² mmol), 107 mg de TPPTS (0,2 mmol) et 0,56 mmol de diméthyl-β-cyclodextrine. La phase organique constituée exclusivement d'undécènoate de méthyle (4 g, 20 mmol) a ensuite été ajoutée. Puis l'autoclave a été mis sous une pression de 50 bar d'un mélange CO/H₂, chauffé à 80°C et agité (1500 t/min).

L'exemple 24 est un essai comparatif effectué dans les mêmes conditions que l'exemple 23, mais sans ajouter de diméthyl-β-cyclodextrine.

Les exemples 25 et 26 ont été réalisés dans les mêmes conditions que l'exemple 23, mais en modifiant le rapport phosphore/rhodium, la pression et la température.

Les conditions réactionnelles et les résultats obtenus sont rassemblés dans le tableau 3.

Le taux de conversion de l'oléfine a été déterminé après 5 heures de réaction.

**Tableau 3**

| Exemple | 23 | 24 | 25 | 26 |
|---|---|---|---|---|
| Rapport phosphore/rhodium | 5 | 5 | 15 | 15 |
| Pression (bar) | 50 | 50 | 50 | 70 |
| Température (°C) | 80 | 80 | 80 | 100 |
| Conversion | 100 | 6 | 75 | 100 |
| Sélectivité | 99 | 99 | 100 | 95 |
| Rapport aldéhydes linéaires/ aldéhydes ramifiés | 1,75 | 4,14 | 2,22 | 2,04 |

La conversion, la sélectivité et le rapport aldéhydes linéaires / aldéhydes ramifiés ont la signification donnée précédemment.

### Exemple 27

Cet exemple a été effectué dans des conditions expérimentales analogues à celle de l'exemple 23, mais en présence d'heptane qui est indispensable pour solubiliser l'oléfine utilisée, le vinylnaphtalène. La réaction a été effectuée dans un autoclave de 25 ml. La phase aqueuse ayant un volume de 9 ml contenait 5,2 mg (2,01.10⁻⁵ mol) de Rh(acac)(CO)₂ avec un rapport TPPTS/Rh = 5, un rapport diméthyl-β-cyclodextrine / rhodium = 14. La phase organique avait un volume de 6 ml. Le rapport oléfine sur rhodium était de 500, la température était de 80°C, la pression (CO/H₂) était de 50 bar. Les résultats sont indiqués dans le tableau 4 ci-dessous.

### Exemple 28

Cet exemple a été effectué dans des conditions expérimentales analogues à celle de l'exemple 1, en utilisant l'acrylate de 2-éthylhexyle comme oléfine fonctionnalisée, en présence de toluène. La réaction a été effectuée dans un autoclave de 100 ml. La phase aqueuse ayant un volume de 30 ml contenait 51,8 mg (0,2.10⁻³ mol) de Rh(acac)(CO)₂ avec un rapport TPPTS/Rh = 10, un rapport diméthyl-β-cyclodextrine / rhodium = 14. La phase organique avait un volume de 40 ml. Le rapport oléfine / rhodium était de 500, la température était de 50°C, la pression (CO/H₂) était de 50 bar. Les résultats sont indiqués dans le tableau 4 ci-dessous.

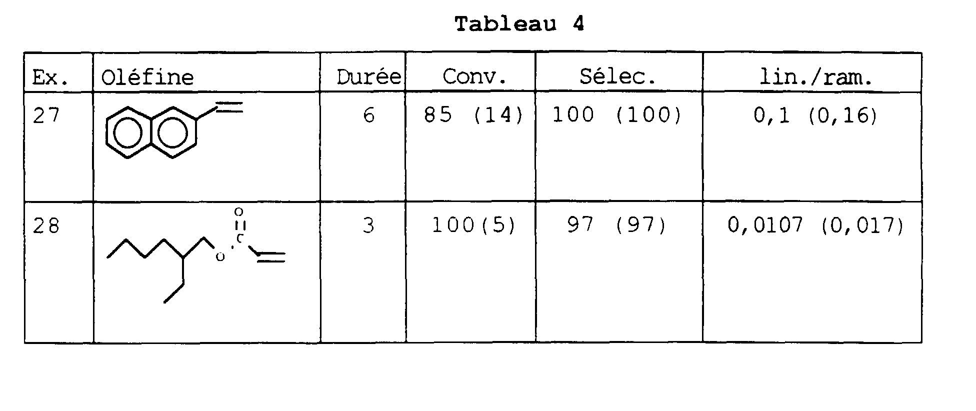

## Revendications

1. Procédé d'hydroformylation d'une oléfine choisie parmi les alcènes représentés par la formule CₙH₂ₙ dans laquelle n est un nombre entier inférieur ou égal à 30 ou parmi les oléfines fonctionnalisées, par un mélange gazeux CO/H₂ en milieu biphasique, consistant à mettre en contact d'une part une solution aqueuse contenant un système catalytique constitué par un complexe de métal de transition hydrosoluble et une phosphine hydrosoluble, et d'autre part une oléfine, à chauffer sous agitation le mélange réactionnel obtenu après l'avoir mis sous une pression de mélange gazeux CO/H₂, le dit procédé étant caractérisé en ce que l'on introduit une cyclodextrine modifiée dans la solution aqueuse contenant le système catalytique, ladite cyclodextrine modifiée étant choisie parmi les α-cyclodextrines, les β-cyclodextrines et les γ-cyclodextrines constituées respectivement de 6, 7 et 8 unités glucosiques, et portant un ou plusieurs substituants différents ou identiques, choisis parmi les radicaux alkyles linéaires ou ramifiés ayant de 1 à 20 atomes de carbone, les radicaux hydroxyalkyles ayant de 1 à 20 atomes de carbone, les radicaux carboxyles, amino, phosphates, éthers, polyéthers, les radicaux comprenant une fonction ester ayant de 1 à 20 atomes de carbone, de préférence 1 à 5 de atomes de carbone, ou parmi les cyclodextrines sous forme polymérisée avec l'épichlorhydrine.

2. Procédé selon la revendication 1, caractérisé en ce que l'on introduit dans la solution aqueuse contenant le système catalytique, un mélange de cyclodextrines modifiées.

3. Procédé selon la revendication 1, caractérisé en ce que l'oléfine est un alcène linéaire ou ramifié ayant une seule double liaison carbone-carbone, en position terminale ou non, ou plusieurs doubles liaisons.

4. Procédé selon la revendication 3, caractérisé en ce l'oléfine est un alcène linéaire ayant de 8 à 30 atomes de carbone.

5. Procédé selon la revendication 1, caractérisé en ce qu'il est mis en oeuvre pour un mélange d'oléfines.

6. Procédé selon la revendication 1, caractérisé en ce que le catalyseur est un complexe d'un métal de transition choisi parmi le rhodium, le ruthénium, le palladium, l'iridium, le cobalt, le platine, le couple platine/étain, le couple platine/fer, avec un ligand phosphoré hydrosoluble.

7. Procédé selon la revendication 6, caractérisé en ce que le catalyseur est un complexe du rhodium ou du cobalt.

8. Procédé selon la revendication 7, caractérisé en ce que le catalyseur est choisi parmi Rh₆(CO)₁₆, Rh₄(CO)₁₂, HRhCO(PR₃)₃ et RhCl(PR₃)₃ (dans lequels R représente un groupement aryle sulfoné, de préférence un groupement phényle sulfoné), Rh(acac)(CO)₂ (acac représentant un radical acétylacétonate), Rh₂Cl₂(CO)₄, les oxydes de rhodium (Rh₂O₃) et les sels de rhodium tels que RhCl₃, Rh(OAc).

9. Procédé selon la revendication 1, caractérisé en ce que la phosphine hydrosoluble est choisie parmi celles qui répondent à l'une des formules générales suivantes : 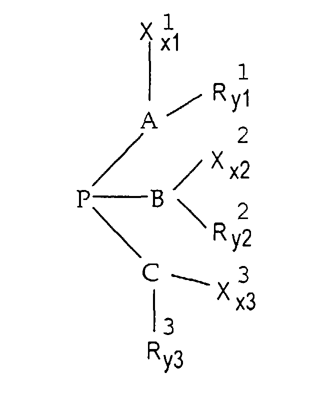 ou 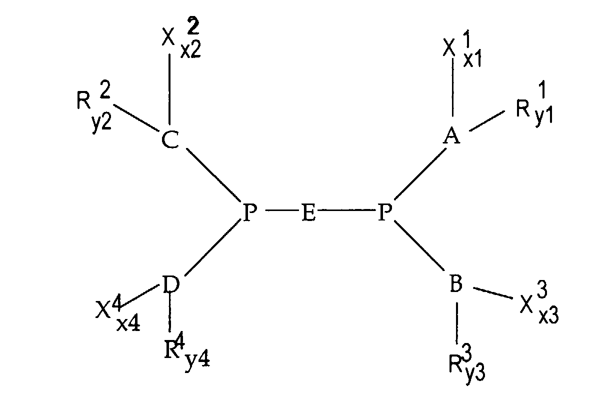 dans lesquelles :
- A, B, C, et le cas échéant D, qui peuvent être identiques ou différents, représentent chacun un groupement aryle choisi parmi les groupements phényles et les groupements naphtyles, ou un groupement alkyle ayant de 1 à 10 atomes de carbone ;
- le cas échéant E représente une chaîne alkyle linéaire ou ramifiée comportant de 1 à 10 atomes de carbone ou une chaîne comportant un ou plusieurs groupements phényles substitués ou non, ou un cycloalcane comprenant éventuellement des substituants alkyles linéaires ou ramifiés ayant de 1 à 6 atomes de carbone ;
- les substituants R¹, R², R³, et le cas échéant R⁴, qui peuvent être identiques ou différents, représentent chacun un groupement choisi parmi l'hydrogène, les radicaux alkyles ayant de 1 à 4 atomes de carbone, les radicaux alkoxy ayant de 1 à 4 atomes de carbone, les atomes d'halogène, les groupes -CN, -NO₂, -OH et -N(Z¹)(Z²), Z¹ et Z² identiques ou différents représentant chacun un radical alkyle ayant de 1 à 5 atomes de carbone ;
- les substituants X¹, X² et X³, et le cas échéant X⁴, qui peuvent être identiques ou différents, représentent chacun un groupement acide carboxylique, un groupement acide sulfonique ou leurs sels ; lorsque X¹, X² ou X³ et le cas échéant X⁴, représentent un sel d'acide carboxylique ou sulfonique, le cation est un cation de métal alcalin ou alcalino-terreux ou un cation ammonium de formule N(T¹T²T³T⁴)⁺ dans laquelle T¹, T², T³, T⁴, qui peuvent être identiques ou différents, représentent chacun un groupe alkyle comportant de 1 à 4 atomes de carbone.
- x1, x2, x3, et le cas échéant x4, sont des nombres identiques ou différents, variant entre 0 et 3 inclus, l'un au moins parmi eux étant égal ou supérieur à 1 ;
- y1, y2, y3, et le cas échéant y4, sont des nombres identiques ou différents variant entre 2 et 5 inclus.

10. Procédé selon la revendication 1, caractérisé en ce que le rapport molaire phosphine / catalyseur est compris entre 1 et 100, de préférence entre 2 et 20.

11. Procédé selon la revendication 1, caractérisé en ce que le rapport molaire cyclodextrine modifiée / catalyseur est compris entre 1 et 100, de préférence entre 2 et 20.

12. Procédé selon la revendication 1, caractérisé en ce que la température de réaction est comprise entre 20° C et 200° C.

13. Procédé selon la revendication 1, caractérisé en ce que la pression est comprise entre 1 et 300 bar, avec une pression partielle de monoxyde de carbone comprise entre 0,5 et 95 bar.

14. Procédé selon la revendication 1, caractérisé en ce que la réaction est effectuée en présence d'un solvant organique non miscible à la phase aqueuse.

15. Procédé selon la revendication 1, caractérisé en ce que la réaction est effectuée en présence d'un solvant polaire non miscible à la phase organique.

## Patentansprüche

1. Verfahren zur Hydroformylierung eines aus Alkenen der Formel CₙH₂ₙ, worin n eine ganze Zahl ≤ 30 ist, oder aus Olefinen mit funktionellen Gruppen ausgewählten Olefins mittels eines CO/H₂-Gasgemischs in einem zweiphasigem Medium, das darin besteht, einerseits eine wässrige Lösung, die ein aus einem wasserlöslichen Übergangsmetallkomplex und einem wasserlöslichen Phosphin bestehendes katalytisches System enthält, und andererseits ein Olefin miteinander in Kontakt zu bringen und das erhaltene Reaktionsgemisch unter Rühren zu erhitzen, nachdem es mit dem CO/H₂-Gasgemisch unter Druck gesetzt wurde, wobei das Verfahren dadurch gekennzeichnet ist, dass ein modifiziertes Cyclodextrin der das katalytische System enthaltenden wässrigen Lösung zugeführt wird, wobei das modifizierte Cyclodextrin aus α-Cyclodextrinen, β-Cyclodextrinen und γ-Cyclodextrinen ausgewählt ist, die jeweils aus 6, 7 und 8 Glucoseeinheiten bestehen, und einen oder mehrere unterschiedliche oder identische Substituenten aufweist, ausgewählt aus unverzweigten oder verzweigten Alkylresten mit 1 bis 20 Kohlenstoffatomen, Hydroxyalkylresten mit 1 bis 20 Kohlenstoffatomen, Carboxyl-, Amino-, Phosphat-, Ether-, Polyetheresten, Resten mit einer Estergruppe mit 1 bis 20 Kohlenstoffatomen, vorzugsweise 1 bis 5 Kohlenstoffatomen, oder aus Cyclodextrinen in mit Epichlorhydrin polymerisierter Form.

2. Verfahren nach Anspruch 1, dadurch gekennzeichnet, dass der das katalytische System enthaltenden wässrigen Lösung ein Gemisch aus modifizierten Cyclodextrinen zugeführt wird.

3. Verfahren nach Anspruch 1, dadurch gekennzeichnet, dass das Olefin ein unverzweigtes oder verzweigtes Alken mit einer einzigen endständigen oder nicht-endständigen Kohlenstoff-Kohlenstoff-Doppelbindung oder mehreren Doppelbindungen ist.

4. Verfahren nach Anspruch 3, dadurch gekennzeichnet, dass das Olefin ein unverzweigtes Alken mit 8 bis 30 Kohlenstoffatomen ist.

5. Verfahren nach Anspruch 1, dadurch gekennzeichnet, dass es mit einem Olefin-Gemisch durchgeführt wird.

6. Verfahren nach Anspruch 1, dadurch gekennzeichnet, dass der Katalysator ein Komplex eines Übergangsmetalls, ausgewählt aus Rhodium, Ruthenium, Palladium, Iridium, Kobalt, Platin, dem Paar Platin/Zinn, dem Paar Platin/Eisen, mit einem wasserlöslichen phoshorhältigen Liganden ist.

7. Verfahren nach Anspruch 6, dadurch gekennzeichnet, dass der Katalysator ein Rhodium- oder Kobalt-Komplex ist.

8. Verfahren nach Anspruch 7, dadurch gekennzeichnet, dass der Katalysator aus Rh₆(CO)₁₆, Rh₄(CO)₁₂, HRhCO(PR₃)₃ und RhCl(PR₃)₃ (worin R für eine sulfonierte Arylgruppe, vorzugsweise eine sulfonierte Phenylgruppe steht), Rh(acac)(CO)₂ (worin acac für einen Acetylacetonat-Rest steht), Rh₂Cl₂(CO)₄, Rhodiumoxiden (Rh₂O₃) und Rhodiumsalzen, wie z.B. RhCl₃, Rh(OAc) ausgewählt ist.

9. Verfahren nach Anspruch 1, dadurch gekennzeichnet, dass das wasserlösliche Phosphin aus jenen ausgewählt ist, die den folgenden allgemeinen Formeln entsprechen: 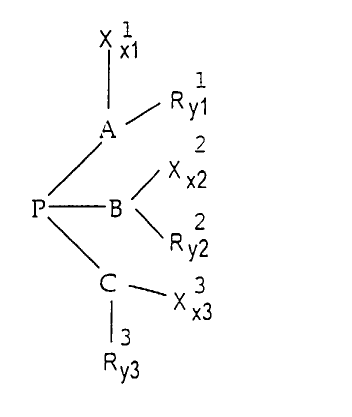 oder 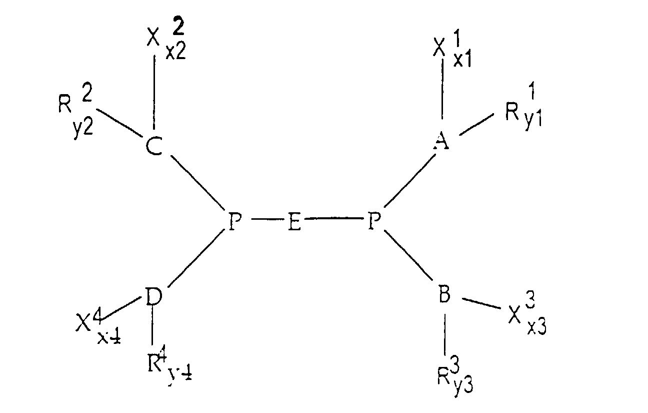 worin:
- A, B, C, und gegebenenfalls D, die.identisch oder voneinander verschieden sein können, jeweils eine Arylgruppe, ausgewählt aus Phenylgruppen und Naphthylgruppen, oder eine Alkylgruppe mit 1 bis 10 Kohlenstoffatomen darstellen;
- E gegebenenfalls eine unverzweigte oder verzweigte Alkylkette mit 1 bis 10 Kohlenstoffatomen oder eine Kette, die eine oder mehrere substituierte oder nicht-substituierte Phenylgruppen umfasst, oder ein Cycloalkan darstellt, das gegebenenfalls unverzweigte oder verzweigte Alkylsubstituenten mit 1 bis 6 Kohlenstoffatomen trägt;
- die Substituenten R¹, R², R³ und gegebenenfalls R⁴, die identisch oder voneinander verschieden sein können, jeweils eine Gruppe darstellen, die aus Wasserstoff, Alkylresten mit 1 bis 4 Kohlenstoffatomen, Alkoxyresten mit 1 bis 4 Kohlenstoffatomen, Halogenatomen und den Gruppen -CN, -NO₂, -OH und -N(Z¹)(Z²) ausgewählt ist, worin Z¹ und Z² identisch oder voneinander verschieden sein können und jeweils einen Alkylrest mit 1 bis 5 Kohlenstoffatomen darstellen;
- die Substituenten X¹, X² und X³ und gegebenenfalls X⁴, die identisch oder voneinander verschieden sein können, jeweils eine Carbonsäuregruppe, eine Sulfonsäuregruppe oder deren Salze darstellen; wobei X¹, X² oder X³ und gegebenenfalls X⁴ ein Carbon- oder Sulfonsäuresalz darstellen, das Kation ein Alkali- oder Erdalkalimetall-Kation oder ein Ammonium-Kation der Formel N(T¹T²T³T⁴)⁺ ist, worin T¹, T², T³, T⁴, die identisch oder voneinander verschieden sein können, jeweils eine Alkylgruppe mit 1 bis 4 Kohlenstoffatomen darstellen;
- x1, x2, x3 und gegebenenfalls x4 identische oder unterschiedliche Zahlen sind die zwischen 0 und 3 (Grenzen eingeschlossen) variieren, wobei zumindest eine davon ≥ 1 ist;
- y1, y2, y3 und gegebenenfalls y4 identische oder unterschiedliche Zahlen sind, die zwischen 2 und 5 (Grenzen eingeschlossen) variieren.

10. Verfahren nach Anspruch 1, dadurch gekennzeichnet, dass das Molverhältnis Phosphin:Katalysator zwischen 1 und 100, vorzugsweise zwischen 2 und 20, liegt.

11. Verfahren nach Anspruch 1, dadurch gekennzeichnet, dass das Molverhältnis modifiziertes Cyclodextrin:Katalysator zwischen 1 und 100, vorzugsweise zwischen 2 und 20, liegt.

12. Verfahren nach Anspruch 1, dadurch gekennzeichnet, dass die Reaktionstemperatur zwischen 20 °C und 200 °C liegt.

13. Verfahren nach Anspruch 1, dadurch gekennzeichnet, dass der Druck zwischen 1 und 300 bar liegt, wobei der Partialdruck von Kohlenmonoxid zwischen 0,5 und 95 bar liegt.

14. Verfahren nach Anspruch 1, dadurch gekennzeichnet, dass die Reaktion in Gegenwart eines organischen Lösungsmittels durchgeführt wird, das mit der wässrigen Phase nicht mischbar ist.

15. Verfahren nach Anspruch 1, dadurch gekennzeichnet, dass die Reaktion in Gegenwart eines polaren Lösungsmittels durchgeführt wird, das mit der organischen Phase nicht mischbar ist.

## Claims

1. A method for the hydroformylation of an olefin chosen from the alkenes represented by the formula CₙH₂ₙ in which n is an integer less than or equal to 30, or chosen from functionalized olefins, by a CO/H₂ gas mixture in a two-phase medium, said method consisting in contacting, on the one hand, an aqueous solution containing a catalytic system consisting of a water-soluble transition metal complex and a water-soluble phosphine, and, on the other hand, an olefin, in heating the reaction mixture obtained with stirring, after it has been placed under a pressure of the CO/H₂ gas mixture, said method being characterised in that a modified cyclodextrin is introduced into the aqueous solution containing the catalytic system, said modified cyclodextrin being chosen from α-cyclo-dextrins, β-cyclodextrins and γ-cyclodextrins consisting respectively of 6, 7 and 8 glucose units and bearing one or more substituents which may be identical or different, chosen from linear or branched alkyl radicals having from 1 to 20 carbon atoms, hydroxyalkyl radicals having from 1 to 20 carbon atoms, carboxylate, amino, phosphate, ether, polyether radicals, radicals comprising an ester function and having from 1 to 5 carbon atoms, or from cyclodextrin in a form polymerised with epichlorhydrine.

2. The method according to claim 1, wherein a mixture of modified cyclodextrins is introduced into the aqueous solution containing the catalytic system.

3. The method according to claim 1, wherein the olefin is a linear or branched alkene having a single carbon-carbon double bond, which may or may not be in the end position, or several double bonds.

4. The method according to claim 3, wherein the olefin is a linear alkene having from 8 to 30 carbon atoms.

5. The method according to claim 1, characterized in that it is carried out with a mixture of olefins.

6. The method according to claim 1, wherein the catalyst is a complex of a transition metal chosen from rhodium, ruthenium, palladium, iridium, cobalt, platinum, the platinum/tin couple or the platinum/iron couple, with a water-soluble phosphorus ligand.

7. The method according to claim 6, wherein the catalyst is a complex of rhodium or of cobalt.

8. The method according to claim 7, wherein the catalyst is chosen from Rh₆(CO)₁₆, Rh₄(CO)₁₂, HRhCO(PR₃)₃ and RhCl(PR₃)₃ (in which R represents an aryl sulfone group, preferably a phenyl sulfone group), Rh(acac)(CO)₂ (acac representing an acetylacetonate radical), Rh₂Cl₂(CO)₄, rhodium oxides (Rh₂O₃) and rhodium salts such as RhCl₃, Rh(OAc)₃.

9. The method according to claim 1, wherein the water-soluble phosphine is chosen from those which correspond to one of the following general formulae: 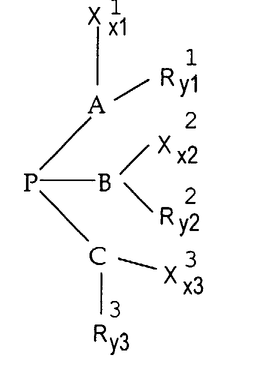 or 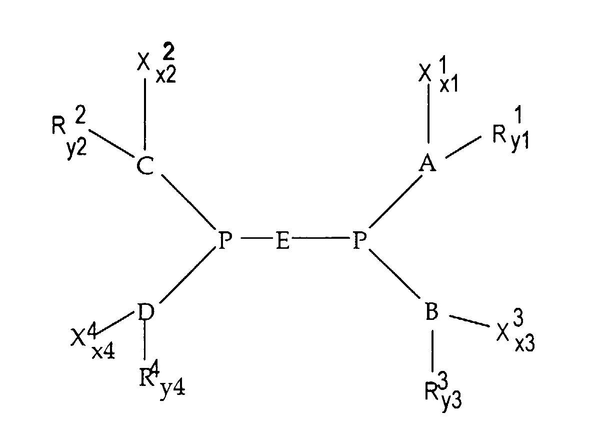 in which:
- A, B, C and, where appropriate, D, which may be identical or different, each represent an aryl group chosen from phenyl groups and naphthyl groups, or an alkyl group having from 1 to 10 carbon atoms;
- where appropriate, E represents a linear or branched alkyl chain containing from 1 to 10 carbon atoms or a chain containing one or more substituted or unsubstituted phenyl groups, or a cycloalkane optionally comprising linear or branched alkyl substituents having from 1 to 6 carbon atoms;
- the substituents R¹, R², R³ and, where appropriate, R⁴, which may be identical or different, each represent a group chosen from hydrogen, alkyl radicals having from 1 to 4 carbon atoms, alkoxy radicals having from 1 to 4 carbon atoms, halogen atoms, -CN, -NO₂, -OH and -N(Z¹)(Z²) groups, Z¹ and Z², which may be identical or different, each representing an alkyl radical having from 1 to 5 carbon atoms;
- the substituents X¹, X², X³ and, where appropriate, X⁴, which may be identical or different, each represent a carboxylic acid group, a sulfonic acid group or salts thereof; when X¹, X², X³ and, where appropriate, X⁴ represent a carboxylic or sulfonic acid salt, the cation is an alkali-metal or alkaline-earth metal cation or an ammonium cation of formula N(T¹T²T³T⁴)⁺ in which T¹, T², T³ and T⁴, which may be identical or different, each represent an alkyl group containing from 1 to 4 carbon atoms;
- x1, x2, x3 and, where appropriate, x4 are identical or different numbers, ranging between 0 and 3 inclusive, at least one of them being greater than or equal to 1;
- y1, y2, y3 and, where appropriate, y4 are identical or different numbers, ranging between 2 and 5 inclusive.

10. The method according to claim 1, wherein the phosphine/catalyst molar ratio is between 1 and 100, preferably between 2 and 20.

11. The method according to claim 1, wherein the modified cyclodextrin/catalyst molar ratio is between 1 and 100, preferably between 2 and 20.

12. The method according to claim 1, wherein the reaction temperature is between 20°C and 200°C.

13. The method according to claim 1, wherein the pressure is between 1 and 300 bar, with a partial pressure of carbon monoxide of between 0.5 and 95 bar.

14. The method according to claim 1, wherein the reaction is carried out in the presence of an organic solvent that is immiscible with the aqueous phase.

15. The method according to claim 1, wherein the reaction is carried out in the presence of a polar solvent that is immiscible with the organic phase.
